(19) Europäisches Patentamt
European Patent Office
Office européen des brevets

(11) **EP 2 184 297 A1**

(12) **EUROPEAN PATENT APPLICATION**

(43) Date of publication:
**12.05.2010 Bulletin 2010/19**

(51) Int Cl.:
*C07K 14/705* (2006.01)   *C12N 15/62* (2006.01)
*C12N 5/10* (2006.01)   *A61K 38/16* (2006.01)

(21) Application number: **08168677.6**

(22) Date of filing: **07.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
• **Hla-G Technologies**
  **69005 Lyon (FR)**
• **COMMISSARIAT A L'ENERGIE ATOMIQUE**
  **75015 Paris (FR)**

(72) Inventors:
• **Favier, Benoit**
  **75019 Paris (FR)**
• **Carosella, Edgardo D.**
  **75016 Paris (FR)**
• **Lemaoult, Joël**
  **77000 Melun (FR)**

(74) Representative: **Becker, Philippe et al**
  **Cabinet Becker & Associés**
  **25, rue Louis Le Grand**
  **75002 Paris (FR)**

(54) **HLA-G polypeptides and pharmaceutical uses thereof**

(57) The present invention relates to novel proteins and pharmaceutical uses thereof. The invention more specifically relates to novel proteins comprising the sequence of an HLA-5 antigen fused to the sequence of a b2 microglobulin. The invention also relates to methods of producing such polypeptides, pharmaceutical compositions comprising the same, as well as their uses for treating various diseases including organ/tissue rejection.

EP 2 184 297 A1

**Description**

The present invention relates to a novel protein and pharmaceutical uses thereof. The invention more specifically relates to a novel fusion protein comprising a domain of an HLA-G5 antigen fused to a B2 microglobulin. The invention also relates to methods of producing such a protein, pharmaceutical compositions comprising the same, as well as their uses for treating various diseases including organ/tissue rejection.

BACKGROUND

Major histocompatibility complex (MHC) antigens are divided up into three main classes, namely class I antigens, class II antigens (HLA-DP, HLA-DQ and HLA-DR), and class III antigens.

Class I antigens comprise conventional antigens, HLA-A, HLA-B and HLA-C, which exhibit 3 globular domains ([alpha]1, [alpha]2 and [alpha]3), as well as unconventional antigens HLA-E, HLA-F, and HLA-G.

HLA-G is a non-classic HLA Class I molecule expressed by extravillous trophoblasts of normal human placenta and thymic epithelial cells. HLA-G antigens are essentially expressed by the cytotrophoblastic cells of the placenta and function as immunomodulatory agents protecting the foetus from the maternal immune system (absence of rejection by the mother). The sequence of the HLA-G gene has been described (e.g., Geraghty et al. Proc. Natl. Acad. Sci. USA, 1987, 84, 9145-9149 ; Ellis; et al., J. Immunol., 1990, 144, 731-735) and comprises 4396 base pairs. This gene is composed of 8 exons, 7 introns and a 3' untranslated end, corresponding respectively to the following domains: exon 1: signal sequence, exon 2: alpha1 extracellular domain, exon 3: alpha2, extracellular domain, exon 4: alpha3 extracellular domain, exon 5: transmembrane region, exon 6: cytoplasmic domain I, exon 7: cytoplasmic domain II (untranslated), exon 8: cytoplasmic domain III (untranslated) and 3' untranslated region.

Seven isoforms of HLA-G have been identified, among which 4 are membrane bound (HLA-G1, HLA-G2, HLA-G3 and HLA-G4) and 3 are soluble (HLA-G5, HLA-G6 and HLA-G7) (see e.g., Carosella et al., Blood 2008, vol. 111, p 4862).

The mature G1 protein isoform comprises the three external domains ($\alpha$1-$\alpha$3), the transmembrane region and the cytoplasmic domain.

The G2 protein isoform does not comprise the $\alpha$2 domain, i.e., the $\alpha$1 and $\alpha$3 domains are directly linked, followed by the transmembrane domain and the cytoplasmic domain.

The G3 protein isoform lacks both the $\alpha$3 and $\alpha$3 domains, i.e., it comprises the $\alpha$1 domain directly linked to the transmembrane domain and the cytoplasmic domain.

The G4 protein isoform lacks the $\alpha$3 domain, i.e., it comprises the $\alpha$1 domain, the $\alpha$2 domain, the transmembrane domain and the cytoplasmic domain.

Soluble HLA-G isoforms all lack the transmembrane and cytoplasmic domains. More specifically:

The G5 protein isoform contains the $\alpha$1, $\alpha$2 and $\alpha$3 domains, as well as an extra C-terminal peptide sequence of 21 amino acid residues encoded by intron 4 (as a result of intron 4 retention after transcript splicing and RNA maturation).

The G6 protein isoform corresponds to the G5 without $\alpha$2, i.e., HLA-G6 contains $\alpha$1 and $\alpha$3 domains, as well as an extra C-terminal peptide sequence of 21 amino acid residues encoded by intron 4 (as a result of intron 4 retention after transcript splicing and RNA maturation).

The G7 protein isoform contains only the alpha1 domain, as well as 2 additional C-terminal amino acid residues encoded by intron2 (as a result of intron 2 retention after transcript splicing and RNA maturation).

All of these isoforms have been described e.g., in Kirszenbaum M. et al., Proc. Natl. Acad. Sci. USA, 1994, 91, 4209-4213; European Application EP 0 677 582; Kirszenbaum M. et al., Human Immunol., 1995, 43, 237-241; Moreau P. et al., Human Immunol., 1995, 43, 231-236).

Previous studies have shown that HLA-G proteins are able to inhibit allogeneic responses such as proliferative T lymphocyte cell response, cytotoxic T lymphocytes mediated cytolysis, and NK cells mediated cytolysis (Rouas-Freiss N. et al., Proc. Natl. Acad. Sci., 1997, 94, 5249-5254 ; Semin Cancer Biol 1999, vol 9, p. 3). As a result, HLA-G proteins have been proposed for treating graft rejection in allogeneic or xenogenic organ/tissue transplantation. HLA-G proteins have also been proposed for the treatment of cancers (EP1 054 688), inflammatory disorders (EP1 189 627) and, more generally, immune related diseases. It has also been proposed to fuse HLA-G proteins to specific ligands in order to target HLA-G to particular cells or tissues (WO2007091078). It should be noted, however, that no results or experimental data have been provided to show that such targeting fusions are active.

At present, it is not clear what conformation is the most active for pharmaceutical purpose, how soluble forms of HLA-G can be used, nor which domains of HLA-G are required for most effective therapy.

SUMMARY OF THE INVENTION

The present invention relates to novel proteins or polypeptides, pharmaceutical compositions comprising the same, and the uses thereof. More specifically, the present invention relates to a novel polypeptide comprising the sequence of an HLA-G5 antigen fused to the sequence of a B2 microglobulin. As shown in the experimental section, this polypeptide is biologically active in vivo, can form dimers, and can produce a strong immune response in a model of graft rejection. This polypeptide thus represents a drug candidate for treating such disorders, as well as other immune-related diseases.

An object of the present invention thus resides in a fusion polypeptide comprising the sequence of a β2 microglobulin fused to the sequence of an HLA-G5 antigen. In a preferred embodiment, the HLA-G5 antigen is a human HLA-G5 antigen. Furthermore, in a preferred embodiment, the sequence of the β2 microglobulin is fused to the sequence of the HLA-G5 antigen through a spacer group and/or is located on the N-terminal part of the polypeptide.

A preferred object of this invention is a polypeptide comprising the following structure:

$$\text{B2M} - \text{SPACER} - \text{HLA-G5}$$

wherein B2M is the sequence of a β2 microglobulin ; SPACER is the sequence of a spacer group comprising preferably from 5 to 20 amino acid residues; and HLA-G5 is the sequence of an HLA-G5 antigen.

A further object of this invention resides in a nucleic acid molecule encoding a polypeptide of this invention.

The invention also relates to a vector comprising a nucleic acid molecule as defined above.

Another object of this invention is a recombinant host cell comprising a nucleic acid molecule or a vector as defined above.

A further object of this invention is a method of producing a polypeptide as defined above, comprising culturing a recombinant host cell of the invention under conditions allowing expression of the nucleic acid molecule, and recovering the polypeptide produced.

The invention further relates to a dimer (e.g., a homodimer or a heterodimer) of a polypeptide of the invention.

The invention also relates to an antibody that specifically binds a fusion polypeptide of this invention or a dimer thereof.

The invention also relates to a pharmaceutical composition comprising a polypeptide as defined above, either as a monomer or as a multimer.

The invention also relates to a pharmaceutical composition comprising a nucleic acid encoding a polypeptide as defined above, or a recombinant cell expressing such a polypeptide.

The invention further relates to such polypeptides or pharmaceutical compositions for treating organ or tissue rejection, inflammatory diseases or auto-immune diseases.

A further objet of this invention also relates to a method of treating organ/tissue rejection, the method comprising administering to a subject in need thereof an effective amount of a polypeptide or composition of this invention. More specifically, the method comprises administering the polypeptide or composition to the subject, prior to, during and/or after tissue/organ transplant.

A further object of this invention is a method of promoting tolerance to graft in a subject, the method comprising administering to a subject in need thereof an effective amount of a polypeptide or composition as defined above.

The invention may be used in any mammalian subject, preferably in human subjects. As will be further disclosed below, the polypeptides of this invention are able to substantially inhibit tissue rejection in vivo following allogeneic or xenogenic transplantation.

LEGEND TO THE FIGURES

    Figure 1: HLA-G5-B2M can form dimers.
    Figure 2: HLA-G5-B2M promotes graft survival in vivo.

DETAILED DESCRIPTION OF THE INVENTION

The present invention relates to fusion polypeptides comprising an HLA-G5 antigen fused to a B2M. The fusion polypeptides of this invention are biologically active and have been shown to effectively inhibit graft rejection in vivo. More specifically, the inventors have found that, by fusing an HLA-G5 antigen to a B2M, biologically active proteins are obtained which exhibit high immune effect. The results presented in this application show that such a polypeptide can promote graft tolerance in vivo very efficiently and therefore represents a novel medicament for treating immune-related disorders, particularly for reducing unwanted or deleterious immune responses in a subject.

A first object of the present invention thus resides in a polypeptide comprising the amino acid sequence of a B2M fused to the amino acid sequence of an HLA-G5 antigen.

Within the context of the present invention, the terms "polypeptide" and "protein" designate, interchangeably, a molecule comprising a polymer of amino acid residues, which may be linked together through amine linkage, or through modified, peptidomimetic linkages. The amino acid residues in said proteins or polypeptides may be either natural amino acid residues, or non-natural or modified amino acid residues. They may be in L and/or D conformation. Also, the polypeptide or protein may be terminally protected and/or modified, e.g., through chemical or physical alteration of lateral functions, for instance.

Within a polypeptide of this invention, the various polypeptide domains are covalently linked together so that they can, most preferably, be produced as a single molecule through recombinant techniques, i.e., they are fused by an amine linkage.

In the proteins of this invention, the B2M sequence is located N-ter of the HLA-G5 sequence and they are linked together through a spacer group, according to the following structure:

$$B2M - SPACER - HLA\text{-}G5$$

wherein B2M is the sequence of a β2 microglobulin ; SPACER is the sequence of a spacer group ; and HLA-G5 is the sequence of an HLA-G5 antigen.

B2 microglobulin ("B2M") is a serum protein of 11,8 KDa which is found in association with the major histocompatibility complex (MHC) class I heavy chain on the surface of nearly all nucleated cells. Beta-2-microglobulin is essential to the cell surface expression of HLA molecules.

A specific example of a sequence of B2M is disclosed in SEQ ID NO: 2 (see amino acid residues 21-119). It should be understood that alternative B2M sequences may be obtained from genebank or from other publications (see, for instance, Genebank number NC 000015 , GeneID:567 , First publication of the sequence: Gussow et al., 1987 [PubMed 3312414]). Furthermore, natural variants of B2M exist, e.g., as a result of polymorphism, which are included in the present application. Also, variants of the above sequences which lack certain (e.g., between 1 and 10, preferably between 1-5, most preferably 1, 2, 3, 4 or 5) amino acid residues, and/or contain certain (e.g., between 1 and 10, preferably between 1-5, most preferably 1, 2, 3, 4 or 5) amino acid substitutions or insertions are also included in the present invention.

The spacer group designates any group (e.g., a peptide) which allows a proper refolding of the polypeptide. The spacer can have a variable length and should preferably be biologically inert. Typically the spacer is a peptide of from 8 to 20 amino acid residues in length, more preferably from 8 to 15, even more preferably from 12 to 15. In a specific embodiment, the spacer group has the sequence (G4S)n, wherein n is 2 or 3.

The HLA-G5 domain contained in the polypeptide comprises the amino acid sequence of an HLA-G5 antigen, preferably of a human HLA-G5 antigen, or variants thereof. As indicated before, the HLA-G5 protein isoform is a soluble protein containing the α1, α2 and α3 domains, as well as an extra C-terminal peptide sequence of 21 amino acid residues encoded by intron 4 (as a result of a modification of the reading frame). HLA-G5 does not contain a transmembrane domain or a cytoplasmic domain.

Within the context of this invention the term "HLA-G5 antigen" therefore designates a polypeptide containing the sequence of α1, α2 and α3 domains of a HLA-G antigen and lacking a transmembrane and a cytoplasmic domain. In a preferred embodiment, the HLA-G5 further comprises an additional the C-terminal peptide sequence of 21 amino acid residues encoded by intron 4 of HLA-G.

A specific example of an HLA-G5 sequence is a sequence consisting of the α1, α2 and α3 domains, as well as the C-terminal peptide sequence of 21 amino acid residues encoded by intron 4.

An example of the amino acid sequence of a human HLA-G5 antigen is provided in SEQ ID NO: 2 (see amino acid residues 135-end). Other HLA-G5 sequences are available on line (see e.g., Fujii, T et al, Journal of Immunology, 1994. PMID: 7989753) or in e.g., US patents No. US5,856,442 and US6,291,659.

It should be understood that natural variants of HLA-G5 exist, e.g., as a result of polymorphism, which are included in the present application. Also, variants of the above sequences which lack certain (e.g., between 1 and 10, preferably between 1 and 5, most preferably 1, 2, 3 or 4) amino acid residues, and/or contain certain (e.g., between 1 and 10, preferably between 1 and 5, most preferably 1, 2, 3 or 4) amino acid substitutions or insertions are also included in the present invention.

A specific example of a fusion polypeptide of the invention is HLA-G5-B2M of SEQ ID NO: 2. In HLA-G5-B2M, the amino acid sequence of human B2M is located on the N-ter side of the polypeptide and is linked to the sequence of a human HLA-G5 antigen through a (G4S)3 spacer group. HLA-G5-B2M further comprises a leader peptide sequence of B2M (amino acid residues 1-20), allowing secretion of the polypeptide.

A further specific polypeptide of this invention is a polypeptide comprising amino acid residues 21-END of SEQ ID NO: 2 (i.e., lacking a leader peptide sequence).

As mentioned in the examples, such a B2M-HLA-G5 polypeptide is able to promote graft tolerance in vivo.

In this respect, the invention demonstrates, for the first time, that HLA-G antigens can be fused to a B2M sequence through specific molecular arrangement to produce fully active biological molecules. The invention thus also resides in a polypeptide having the following structure:

$$B2M - SPACER - HLA\text{-}G\ \text{antigen}$$

$$\text{N-ter} \hspace{8cm} \text{C-ter}$$

wherein B2M is the sequence of a β2 microglobulin as defined above; SPACER is a spacer group as defined above ; and HLA-G is the sequence of an HLA-G antigen. The sequence of the HLA-G antigen typically comprises the sequence of at least the α1 domain of an HLA-G antigen, preferably of a human HLA-G antigen.

A further object of this invention is a dimer of a polypeptide as defined above. The dimer may be a homodimer, e.g., between two identical polypeptides, or a heterodimer, e.g., a dimer comprising at least one polypeptide of this invention.

The polypeptides of this invention can be obtained using techniques known per se in the art, such as artificial synthesis, recombinant techniques, and/or combinations thereof. In a typical embodiment, as illustrated in the examples, the polypeptide is produced by recombinant techniques, starting from a chimeric coding polynucleotide.

In this respect, a further object of this invention is a nucleic acid molecule encoding a polypeptide as defined above. The nucleic acid may be e.g., RNA or DNA, single- or double-stranded. It may be produced by techniques known per se in the art, such as genetic engineering, chemical or enzymatic synthesis, etc. In a particular embodiment, the nucleic acid further comprises a sequence encoding a leader peptide for secretion, operably linked to the sequence encoding the polypeptide. As a result, expression of such a nucleic acid leads to the secretion of the polypeptide by the selected host cell. The leader peptide may by of various origins, such as from human or mammalian genes, e.g., B2M, interleukin, HLA-G, etc. A specific example of a nucleic acid of this invention comprises SEQ ID NO: 1 (or nucleotide residues 61-END thereof, i.e., without the leader sequence).

A further object of this invention also resides in a vector comprising a nucleic acid as defined above. The vector may be a cloning and/or expression vector, such as a plasmid, cosmid, phage, a viral vector, an artificial chromosome, etc. Specific examples of such vectors include pFUSE plasmids, pUC plasmids, pcDNA plasmids, pBR plasmids, retroviral vectors, adenoviral vectors, baculoviral vectors, lambda phage vectors, etc. The vector may comprise regulatory sequences, such as a promoter, a terminator, an origin of replication, etc. The vector may be used to produce polypeptides of this invention in vitro, by recombinant techniques, or directly in vivo, in gene therapy approaches.

A further object of this invention is a recombinant host cell comprising a nucleic acid or a vector as defined above. The host cell may be prokaryotic or eukaryotic. Examples of prokaryotic hosts include any bacteria, such as E. coli. Examples of eukaryotic cells include yeasts, fungi, mammalian cells, plant cells or insect cells. Recombinant cells of this invention may be prepared by transformation techniques known per se in the art, such as transfection, lipofection, electroporation, protoplast transformation, etc. These cells may be maintained and cultured in any suitable culture media.

Recombinant cells of this invention can be used e.g., to produce polypeptides of this invention in vitro or ex vivo, or as cell therapy products, to produce the polypeptides in vivo.

In this respect, an object of this invention also resides in a method of producing a polypeptide as disclosed above, the method comprising culturing a recombinant host cell of the invention under conditions allowing expression of the nucleic acid molecule, and recovering the polypeptide produced. The polypeptide may be recovered and/or purified using techniques known per se in the art, such as centrifugation, filtration, chromatographic techniques, etc.

Upon production, the polypeptides of this invention may be modified to improve their properties, for instance to improve their pharmaco-kinetic properties. In this respect, they may be modified to increase their stability or resistance to protease, such as by adding terminal protecting groups (e.g., amide, ester). They may also be coated on a carrier support to increase the polypeptide density.

A further object of this invention is a pharmaceutical composition comprising a polypeptide as defined above and, preferably, a pharmaceutically acceptable excipient or carrier.

A further object of this invention is a pharmaceutical composition comprising a nucleic acid as defined above and, preferably, a pharmaceutically acceptable excipient or carrier.

A further object of this invention is a pharmaceutical composition comprising a recombinant cell as defined above and, preferably, a pharmaceutically acceptable excipient or carrier.

Suitable excipients or carriers include any pharmaceutically acceptable vehicle such as buffering agents, stabilizing agents, diluents, salts, preservatives, emulsifying agents, sweeteners, etc. The excipient typically comprises an isotonic aqueous or non aqueous solution, which may be prepared according to known techniques. Suitable solutions include buffered solutes, such as phosphate buffered solution, chloride solutions, Ringer's solution, and the like. The pharmaceutical preparation is typically in the form of an injectable composition, preferably a liquid injectable composition, although other forms may be contemplated as well, such as tablets, gelules, capsules, syrups, etc. The compositions of this invention may be administered by a number of different routes, such as by systemic, parenteral, oral, rectal, nasal or vaginal route. They are preferably administered by injection, such as intravenous, intraarterial, intramuscular, intraperitoneal, or subcutaneous injection. Transdermal administration is also contemplated. The specific dosage can be adjusted by the skilled artisan, depending on the pathological condition, the subject, the duration of treatment, the presence of other active ingredients, etc. Typically, the compositions comprise unit doses of between 10ng and 1 mg of fusion polypeptide, more preferably between 10 ng and 100mg, even more preferably between 10$\mu$g and 100 mg.

The compositions of the present invention are preferably administered in effective amounts, i.e., in amounts which are, over time, sufficient to at least reduce or prevent disease progression. In this regard, the compositions of this invention are preferably used in amounts which allow the reduction of a deleterious or unwanted immune response in a subject.

As mentioned above, the polypeptides of this invention have strong immune-regulatory activity and may be used to treat a variety of disease conditions associated with abnormal or unwanted immune response. More specifically, the polypeptides of this invention are suitable for treating immune-related disorders such as, particularly, organ or tissue rejection, inflammatory diseases or auto-immune diseases.

As disclosed in the experimental section, the polypeptides of this invention can substantially inhibit allogeneic or xenogenic graft rejection in vivo.

An object of the present invention thus resides in a polypeptide or composition as disclosed above for treating graft rejection.

A further object of this invention resides in a method of treating graft rejection in a subject, the method comprising administering to a subject in need thereof an effective amount of a composition as disclosed above.

The term treating designates for instance the promotion of the graft tolerance within the receiving subject. The treatment can be performed prior to, during and/or after the graft, and may be used as an alternative therapy to existing immunosuppressive agents or, as a combined therapy with actual immunosuppressive agents. The invention is applicable to allogenic, semi-allogenic or even xenogenic transplantation, and may be used for any type of transplanted organs or tissues including, without limitation, solid tissues, liquid tissues or cells, including heart, skin, kidney, liver, lung, liver-kidney, etc.

A further object of this invention is an improved method for transplanting an organ or tissue in a subject, the improvement comprising administering to the subject, prior to, during and/or after transplantation, an effective amount of a composition as disclosed above.

A further object of this invention is a method for promoting graft tolerance in a subject, the method comprising administering to the subject, prior to, during and/or after transplantation, an effective amount of a composition as disclosed above.

A further object of this invention is a method for reducing graft rejection in a subject, the method comprising administering to the subject, prior to, during and/or after transplantation, an effective amount of a composition as disclosed above.

A further object of the present invention resides in a polypeptide or composition as disclosed above for treating an auto-immune disease. The invention also resides in a method of treating an autoimmune disease in a subject, the method comprising administering to a subject in need thereof an effective amount of a composition as disclosed above. The autoimmune disease may be Rheumatoid arthritis, Crohn's disease or multiple sclerosis. In such disease conditions, the invention allows to reduce the deleterious immune response which is responsible for the pathology.

Another object of the present invention resides in a polypeptide or composition as disclosed above for treating an inflammatory disease.

A further object of this invention resides in a method of treating an inflammatory disease in a subject, the method comprising administering to a subject in need thereof an effective amount of a composition as disclosed above.

It should be understood that the amount of the composition actually administered shall be determined and adapted by a physician, in the light of the relevant circumstances including the condition or conditions to be treated, the exact composition administered, the age, weight, and response of the individual patient, the severity of the patient's symptoms, and the chosen route of administration. Therefore, the above dosage ranges are intended to provide general guidance and support for the teachings herein, but are not intended to limit the scope of the invention.

Further aspects and advantages of this invention will be disclosed in the following examples, which should be considered as illustrative and not limiting the scope of this application.

<u>EXAMPLES</u>

**Materials and Methods**

<u>Amplification by PCR</u>

PCR were performed on a GeneAmp PCR System 9600 (Perkin Elmer) in a final volume of 50$\mu$l containing 20ng of DNA, 200nM of each primer, 200$\mu$M of dNTP (Invitrogen), 2.5$\mu$l of PCR Buffer 10X (Perkin Elmer), 2,5 Unit of Taq polymerase (Perkin Elmer) and 27$\mu$l of water.

Program used was the following :

DNA denaturation during 5 minutes at 94°C followed by 30 cycles of:

30 seconds at 94°C
30 seconds at 58°C
1 minute at 72°C

At the end of the last cycle a 5 minutes step at 72°C was performed

<u>Vector</u>

pFUSE-hFcl and pFuse-mFc2 vector were both purchased from the company InvivoGen.

<u>Enzymatic digestions</u>

Enzymes restriction digestions were performed as recommended by the manufacturer (invitrogen). Typically, digestions were performed for 1 hour at 37°C with 1$\mu$g of DNA and 5 unit of restriction enzymes in the adequate buffer.

<u>Ligations</u>

Ligation of PCR fragments into expression vector were performed with the T4 DNA ligase from Promega as recommended by the manufacturer. For HLA-G5-beta-2 microglobulin construction ligation of PCR fragment into pcDNA 3.1 D/V.5-His-Topo (Invitrogen) was performed directly with the 3.1 Directional TOPO® Expression Kit (Invitrogen)

<u>Plasmid purification</u>

Plasmid purification were performed with the GenElute™ Plasmid Midiprep (Sigma) as recommended by the manufacturer.

<u>Protein production</u>

For production of the fusion protein, HEK293T or HELA cells were transfected by the diverse constructs with the lipofectamine method (invitrogen) and kept at 37°C, 5%CO2 in DMEM (Dulbco's Modified EagleMedium) supplemented with 10% foetal calf serum and 0.3M glutamine. After 48 hours supernatant were harvested, filtrated through 0.2 $\mu$M filter and then used for experiments or to prepare stocks.

**Example 1: Cloning and synthesis of HLA-G5-B2M**

Beta-2-microglobulin fused to $\alpha$1, $\alpha$2 and $\alpha$3 domain was amplified by PCR using plasmid pFUSE-hFc1-HLAG1-B2M as template (unpublished) with primers « B2Msig TOPO sens » 5' CACCATGTCTCGCTCCGTGGCC (SEQ ID NO: 3) and «alpha3-i4-Xho-**Stop** anti sens » : 5' ATC **TTA** ACT CGA GAG GTC TTC AGA GAG GCT CCT GCT TTC CCT AAC AGA CAT GAT GCC TCC ATC TCC CTC CTT ACT CCA TCT CAG CAT GAG 3' (SEQ ID NO: 4) that contains intron 4 sequence from HLA-G5. The PCR fragment was then ligated into the pcDNA 3.1 D/V.5-His-Topo vector (Invitrogen) using 3.1 Directional TOPO® Expression Kit (Invitrogen).

The resulting cDNA sequence is described in SEQ ID NO: 1. The amino acid sequence of the protein is described in SEQ ID NO: 2.

The protein was produced as disclosed in the materials and methods.

**Example 2: HLA-G5-B2M forms dimers**

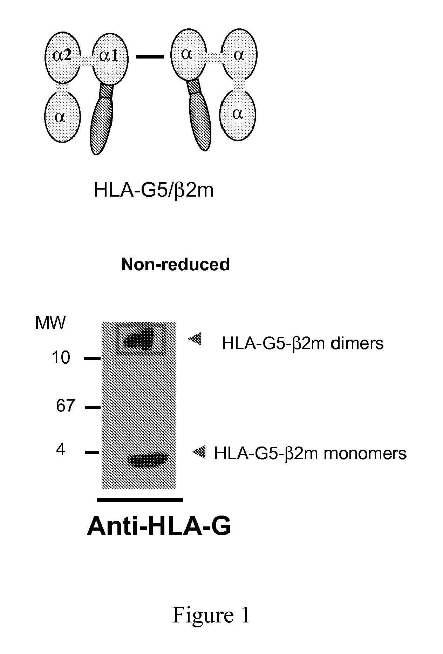

Figure 1 represents HLA-G5-beta-2-microglobulin dimers (upper band) and monomers (lower band) protein migration by PolyAcrylamide Gel Electrophoresis. HLA-G5-beta-2-microglobulin protein present in supernatant was immunoprecipitated with Protein G sepharose beads (GE Healthcare) previously coated with anti-HLA-G5 antibody (MEMG/09). Immunoprecipitates were washed three times with PBS IX. Proteins were then eluted by incubation with sample buffer in non-reducing condition, boiling, electrophoresed on polyacrylamide gels and transferred onto Hybond ECL nitrocellulose membranes (Amersham Pharmacia Biosciences). Following incubation with 5% non-fat milk in PBS 1X, the membrane was incubated overnight with anti-HLA-G (4H84) antibody and revealed using HorseRadish peroxydase-conjugated goat anti-mouse secondary antibody. Membranes were revealed with ECL detection system (Amersham Pharmacia Biosciences).

The results presented demonstrate the ability of fusion proteins of this invention to form dimers.

**Example 3: HLA-G5-B2M promotes survival of allogeneic skin transplant in vivo**

Material

Sulfate latex beads 4%w/v 5$\mu$m (Invitrogen)
AffiniPure Coat Anti-mouse IgG Fc Fragment 1.8mg/ml (Jackson ImmunoResearch)
AffiniPure Coat Anti-human IgG Fc Fragment 1.3mg/ml (Jackson ImmunoResearch)
HeLa Negative Control
HLAG5-b2m 1.5 $\mu$g/ml

Method

For every HLA-G/Fc fusion protein, $10^8$ Sulfate latex beads were coated with 20$\mu$g/ml AffiniPure Coat Anti-mouse (or anti-human) IgG Fc Fragment 2hr at 37°C followed by 2hr incubation with BSA (2 mg/ml). After washing, the beads were incubated with 0.5$\mu$g/ml of HLA-G/Fc fusion proteins at 4°C for 16hr. Subsequently, the beads were washed 2 times by Ix PBS. 5ml of HLA-G/Fc fusion proteins (1$\mu$g/ml) was used for 5x $10^6$ sulfate latex beads. As a negative control, sulfate latex beads were prepared in an identical manner except that 1xPBS or HeLa Negative Control was used rather than HLA-G/Fc fusion proteins. Sulfate latex beads ($5 \times 10^6$) were injected intraperitoneal (i.p.) on the day before skin grafting.

Specific pathogen-free C57BL/6 (H-2b) mice and ILT4-transgenic mice (H-2b) (8-10 weeks of age) were used as skin graft recipients throughout the study. Recipient mice received HLA-G-coupled micro spheres, Donor skin was from MHC class II-disparate B6.CH-2bm12 (bm12, H-2b) mice. Allogeneic skin grafts have been performed by standard methods. Briefly, skin (1.0 cm$^2$) from the tail of donor mice (12-14 weeks old) was grafted onto the flank of recipient, anesthetized mice. The graft was covered with gauze and plaster, which was removed on day 10. Grafts were scored daily until rejection (defined as 80% of grafted tissue becoming necrotic and reduced in size). All skin grafting survival data were tested by Kaplan Meier Survival Analysis.

Results

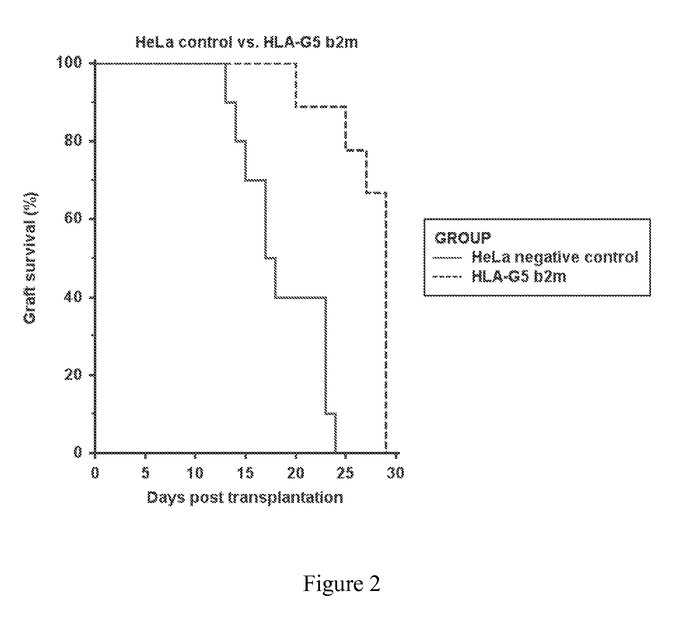

The results are depicted on Figure 2. They show that HLA-G5-B2M was able to substantially improve graft tolerance in vivo. It should be noted that each day of graft survival in the model corresponds to approximately at least one month of graft survival in human subjects.

## SEQUENCE LISTING

SEQ ID NO: 1 : cDNA

seq signal B2m/**Beta2m**/Linker/<u>Gα1/Gα2/Gα3/**intron4**</u>

ATG TCT CGC TCC GTG GCC TTA GCT GTG CTC GCG CTA CTC TCT CTT
TCT GGC CTG GAG GCT **ATC CAG CGT ACT CCA AAG ATT CAG GTT TAC**
**TCA CGT CAT CCA GCA GAG AAT GGA AAG TCA AAT TTC CTG AAT TGC**
**TAT GTG TCT GGG TTT CAT CCA TCC GAC ATT GAA GTT GAC TTA CTG**
**AAG AAT GGA GAG AGA ATT GAA AAA GTG GAG CAT TCA GAC TTG TCT**
**TTC AGC AAG GAC TGG TCT TTC TAT CTC TTG TAC TAC ACT GAA TTC**
**ACC CCC ACT GAA AAA GAT GAG TAT GCC TGC CGT GTG AAC CAT GTG**
**ACC TTG TCA CAG CCC AAG ATA GTT AAG TGG GAT CGA GAC ATG** GGA
GGT GGC GGA TCC GGA GGT GGC GGA TCC GGA GGT GGC GGA TCC <u>GGC</u>
<u>TCC CAC TCC ATG AGG TAT TTC AGC GCC GCC GTG TCC CGG CCC GGC</u>
<u>CGC GGG GAG CCC CGC TTC ATC GCC ATG GGC TAC GTG GAC GAC ACG</u>
<u>CAG TTC GTG CGG TTC GAC AGC GAC TCG GCG TGT CCG AGG ATG GAG</u>
<u>CCG CGG GCG CCG TGG GTG GAG GAG GAG GGG CCG GAG TAT TGG GAA</u>
<u>GAG GAG ACA CGG AAC ACC AAG GCC CAC GCA CAG ACT GAC AGA ATG</u>
<u>AAC CTG CAG ACC CTG CGC GGC TAC TAC AAC CAG AGC GAG GCC AGT</u>
<u>TCT CAC ACC CTC CAG TGG ATG ATT GGC TGC GAC CTG GGG TCC GAC</u>
<u>GGA CGC CTC CTC CGC GGG TAT GAA CAG TAT GCC TAC GAT GGC AAG</u>
<u>GAT TAC CTC GCC CTG AAC GAG GAC CTG CGC TCC TGG ACC GCA GCG</u>
<u>GAC ACT GCG GCT CAG ATC TCC AAG CGC AAG TGT GAG GCG GCC AAT</u>
<u>GTG GCT GAA CAA AGG AGA GCC TAC CTG GAG GGC ACG TGC GTG GAG</u>
<u>TGG CTC CAC AGA TAC CTG GAG AAC GGG AAG GAG ATG CTG CAG CGC</u>
<u>GCG GAC CCC CCC AAG ACA CAC GTG ACC CAC CAC CCT GTC TTT GAC</u>
<u>TAT GAG GCC ACC CTG AGG TGC TGG GCC CTG GGC TTC TAC CCT GCG</u>
<u>GAG ATC ATA CTG ACC TGG CAG CGG GAT GGG GAG GAC CAG ACC CAG</u>
<u>GAC GTG GAG CTC GTG GAG ACC AGG CCT GCA GGG GAT GGA ACC TTC</u>
<u>CAG AAG TGG GCA GCT GTG GTG GTG CCT TCT GGA GAG GAG CAG AGA</u>
<u>TAC ACG TGC CAT GTG CAG CAT GAG GGG CTG CCG AGC CCC CTC ATG</u>
<u>CTG AGA TGG AGT AAG GAG GGA GAT GGA GGC ATC ATG TCT GTT AGG</u>
<u>GAA AGC AGG AGC CTC TCT GAA GAC CTC</u>

SEQ ID NO: 2 : Amino acid sequence of HLA-G5/β2m.

```
seq signal B2m: residues 1-20
Beta2m: residues 21-119
Linker: residues 120-134
Gα1/Gα2/Gα3/intron4: residues 135-END
```

**M** S R S V A L A V L A L L S L S G L E A I Q R T P K I Q V Y

S R H P A E N G K S N F L N C Y V S G F H P S D I E V D L L

K N G E R I E K V E H S D L S F S K D W S F Y L L Y Y T E F

T P T E K D E Y A C R V N H V T L S Q P K I V K W D R D M G

G G G S G G G G S G G G G S G S H S M R Y F S A A V S R P G

R G E P R F I A M G Y V D D T Q F V R F D S D S A C P R M E

P R A P W V E E E G P E Y W E E E T R N T K A H A Q T D R M

N L Q T L R G Y Y N Q S E A S S H T L Q W M I G C D L G S D

G R L L R G Y E Q Y A Y D G K D Y L A L N E D L R S W T A A

D T A A Q I S K R K C E A A N V A E Q R R A Y L E G T C V E

W L H R Y L E N G K E M L Q R A D P P K T H V T H H P V F D

Y E A T L R C W A L G F Y P A E I I L T W Q R D G E D Q T Q

D V E L V E T R P A G D G T F Q K W A A V V V P S G E E Q R

Y T C H V Q H E G L P E P L **M** L R W K A V A S K E G D G G I

**M** S V R E S R S L S E D L

## Claims

1. A polypeptide comprising, in the N-ter -> C-ter orientation, the amino acid sequence of a β2 microglobulin, a spacer group and the amino acid sequence of an HLA-G5 antigen.

2. The polypeptide of claim 1, wherein the β2 microglobulin is a human b2 microglobulin.

3. The polypeptide of claim 1 or 2, wherein the β2 microglobulin comprises amino acid residues 21-119 of SEQ ID NO: 2.

4. The polypeptide of any one of the preceding claims, wherein the HLA-G5 antigen is a human HLA-G5 antigen.

5. The polypeptide of any one of the preceding claims, wherein the HLA-G5 antigen is selected from:

    a) the amino acid sequence of the α1, α2 and α3 domains of an HLA-G antigen;
    b) the amino acid sequence of the α1, α2 and α3 domains and of the 21 amino acid residues encoded by intron 4 of an HLA-G antigen;
    c) the amino acid residues 135-END of SEQ ID NO: 2 ; and
    d) an amino acid sequence as defined in any one of a) to c) having from 1 to 5 amino acid deletion, substitution or insertion.

6. The polypeptide of anyone of the preceding claims, wherein the spacer group is a peptide of 8 to 20 amino acid residues, preferably of (G4S)n sequence, wherein n is 2 or 3.

7. A polypeptide HLA-G5-B2M comprising SEQ ID NO: 2 or amino acid residues 21-END thereof.

8. A nucleic acid molecule encoding a polypeptide of any one of claims 1 to 7.

9. A vector comprising a nucleic acid molecule of claim 8.

10. A recombinant host cell comprising a nucleic acid molecule of claim 8 or a vector of claim 9.

11. A method of producing a polypeptide of any one of claims 1 to 7, comprising culturing a recombinant host cell of claim 10 under conditions allowing expression of the nucleic acid molecule, and recovering the polypeptide produced.

12. A dimer of a polypeptide of any one of claims 1 to 7.

13. A pharmaceutical composition comprising a polypeptide of any one of claims 1 to 7.

14. The pharmaceutical composition of claim 13, for treating organ or tissue rejection.

15. The pharmaceutical composition of claim 14, for treating an inflammatory disease or an auto-immune disease.

HLA-G5/β2m

**Non-reduced**

MW

10 —

67 —

4 —

◄ HLA-G5-β2m dimers

◄ HLA-G5-β2m monomers

**Anti-HLA-G**

Figure 1

Figure 2

Europäisches
Patentamt

European
Patent Office

Office européen
des brevets

# EUROPEAN SEARCH REPORT

**Application Number**

EP 08 16 8677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 01/72768 A (TECHNION RES & DEV FOUNDATION [IL]; REITER YORAM [IL]) 4 October 2001 (2001-10-04) * the whole document * | 1-15 | INV. C07K14/705 C12N15/62 C12N5/10 A61K38/16 |
| Y | WO 02/22784 A (UNIV KANSAS MEDICAL CT [US]; HUNT JOAN S [US]; MORALES PEDRO J [US]; P) 21 March 2002 (2002-03-21) * the whole document * especially page 32, second | 1-15 | |
| D,Y | WO 00/78337 A (COMMISSARIAT ENERGIE ATOMIQUE [FR]; ARACTINGI SELIM [FR]; CAROSELLA ED) 28 December 2000 (2000-12-28) * the whole document * especially example 1 and 2 | 1-15 | |
| Y | GONEN-GROSS TSUFIT ET AL: "The CD85J/leukocyte inhibitory receptor-1 distinguishes between conformed and beta(2)-microglobulin-free HLA-G molecules" October 2005 (2005-10), JOURNAL OF IMMUNOLOGY, VOL. 175, NR. 8, PAGE(S) 4866-4874 , XP002522099 ISSN: 0022-1767 * the whole document * | 1-15 | TECHNICAL FIELDS SEARCHED (IPC) C07K |

-/--

The present search report has been drawn up for all claims

| Place of search | Date of completion of the search | Examiner |
|---|---|---|
| The Hague | 1 April 2009 | Van der Schaal, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 08 16 8677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LEE L ET AL: "Functional cell surface expression by a recombinant single-chain class I major histocompatibility complex molecule with a cis-active B2-microglobulin domain" EUROPEAN JOURNAL OF IMMUNOLOGY, WILEY - V C H VERLAG GMBH & CO. KGAA, DE, vol. 24, 1 January 1994 (1994-01-01), pages 2633-2639, XP002955722 ISSN: 0014-2980 * the whole document * | 1-15 | |
| Y | LIANG SIYUAN ET AL: "Modulation of dendritic cell differentiation by HLA-G and ILT4 requires the IL-6-STAT3 signaling pathway" PROCEEDINGS OF THE NATIONAL ACADEMY OF SCIENCES OF THE UNITED STATES OF AMERICA, vol. 105, no. 24, June 2008 (2008-06), pages 8357-8362, XP002522096 ISSN: 0027-8424 * abstract * | 12 | |
| Y | LE ROND SOLENE ET AL: "Evidence to support the role of HLA-G5 in allograft acceptance through induction of immunosuppressive/regulatory T cells" JOURNAL OF IMMUNOLOGY, vol. 176, no. 5, March 2006 (2006-03), pages 3266-3276, XP002522097 ISSN: 0022-1767 * the whole document * | 13-15 | TECHNICAL FIELDS SEARCHED (IPC) |

-/--

The present search report has been drawn up for all claims

| Place of search | Date of completion of the search | Examiner |
|---|---|---|
| The Hague | 1 April 2009 | Van der Schaal, C |

EPO FORM 1503 03.82 (P04C01)

Europäisches
Patentamt
European
Patent Office
Office européen
des brevets

**EUROPEAN SEARCH REPORT**

Application Number

EP 08 16 8677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROUAS-FREISS N ET AL: "THE IMMUNOTOLERANCE ROLE OF HLA-G" SEMINARS IN CANCER BIOLOGY, SAUNDERS SCIENTIFIC PUBLICATIONS, PHILADELPHIA, PA, US, vol. 9, 1 January 1999 (1999-01-01), pages 3-12, XP002908345 ISSN: 1044-579X * page 11, left-hand column, last paragraph * ----- | 13-15 | |
| D,Y | CAROSELLA EDGARDO D ET AL: "Beyond the increasing complexity of the immunomodulatory HLA-G molecule" BLOOD, AMERICAN SOCIETY OF HEMATOLOGY, US, vol. 111, no. 10, 15 May 2008 (2008-05-15), pages 4862-4870, XP008104444 ISSN: 0006-4971 [retrieved on 2008-03-11] * page 4863, right-hand column, paragraph 2 - page 4865 * ----- | 12-15 | |
| Y | ROUAS-FREISS NATHALIE ET AL: "HLA-G in transplantation: a relevant molecule for inhibition of graft rejection?" AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS JAN 2003, vol. 3, no. 1, January 2003 (2003-01), pages 11-16, XP002522098 ISSN: 1600-6135 * the whole document * ----- | 13,14 | TECHNICAL FIELDS SEARCHED (IPC) |

The present search report has been drawn up for all claims

| Place of search | Date of completion of the search | Examiner |
|---|---|---|
| The Hague | 1 April 2009 | Van der Schaal, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 08 16 8677

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2009

| Patent document cited in search report | | Publication date | Patent family member(s) | | Publication date |
|---|---|---|---|---|---|
| WO 0172768 | A | 04-10-2001 | AU | 785493 B2 | 03-01-2008 |
| | | | AU | 3953401 A | 08-10-2001 |
| | | | CA | 2404489 A1 | 04-10-2001 |
| | | | EP | 1294748 A2 | 26-03-2003 |
| | | | JP | 2004503213 T | 05-02-2004 |
| | | | US | 2003003535 A1 | 02-01-2003 |
| | | | US | 2004086960 A1 | 06-05-2004 |
| | | | ZA | 200207515 A | 21-07-2004 |
| WO 0222784 | A | 21-03-2002 | AU | 9277301 A | 26-03-2002 |
| | | | CA | 2425186 A1 | 21-03-2002 |
| | | | EP | 1328290 A2 | 23-07-2003 |
| WO 0078337 | A | 28-12-2000 | AT | 251911 T | 15-11-2003 |
| | | | CA | 2377519 A1 | 28-12-2000 |
| | | | DE | 60005952 D1 | 20-11-2003 |
| | | | DE | 60005952 T2 | 29-07-2004 |
| | | | DK | 1189627 T3 | 16-02-2004 |
| | | | EP | 1189627 A1 | 27-03-2002 |
| | | | ES | 2206286 T3 | 16-05-2004 |
| | | | FR | 2794977 A1 | 22-12-2000 |
| | | | JP | 2003508351 T | 04-03-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0677582 A
- EP 1054688 A
- EP 1189627 A
- WO 2007091078 A
- US 5856442 A
- US 6291659 B

**Non-patent literature cited in the description**

- **Geraghty et al.** *Proc. Natl. Acad. Sci. USA,* 1987, vol. 84, 9145-9149
- **Ellis et al.** *J. Immunol.,* 1990, vol. 144, 731-735
- **Carosella et al.** *Blood,* 2008, vol. 111, 4862
- **Kirszenbaum M. et al.** *Proc. Natl. Acad. Sci. USA,* 1994, vol. 91, 4209-4213
- **Kirszenbaum M. et al.** *Human Immunol.,* 1995, vol. 43, 237-241
- **Moreau P. et al.** *Human Immunol.,* 1995, vol. 43, 231-236
- **Rouas-Freiss N. et al.** *Proc. Natl. Acad. Sci.,* 1997, vol. 94, 5249-5254
- *Semin Cancer Biol,* 1999, vol. 9, 3
- **Gussow et al.** *PubMed 3312414,* 1987
- **Fujii, T et al.** *Journal of Immunology,* 1994